# EUROPEAN PATENT SPECIFICATION

(11) **EP 3 801 706 B1**
(45) Date of publication and mention of the grant of the patent: **16.02.2022**
(21) Application number: 19726695.0
(22) Date of filing: 28.05.2019
(51) Int. Cl.: A61M 11/00, A61M 15/00

(54) **A SYSTEM FOR GENERATING A DROPLET OUTPUT AND A METHOD OF MONITORING CLEANING**
SYSTEM ZUR ERZEUGUNG EINER TRÖPFCHENABGABE UND VERFAHREN ZUR ÜBERWACHUNG DER REINIGUNG
SYSTÈME DE GÉNÉRATION D'UNE SORTIE DE GOUTTELETTE ET PROCÉDÉ DE SURVEILLANCE DE NETTOYAGE

(30) Priority: 05.06.2018 US 201862680729 P; 19.06.2018 EP 18178527
(43) Date of publication of application: 14.04.2021
(73) Proprietor: Koninklijke Philips N.V., 5656 AG Eindhoven (NL)
(72) Inventor: VON HOLLEN, Dirk, Ernest, 5656 AE Eindhoven (NL); PRITCHARD, John, Nigel, 5656 AE Eindhoven (NL)
(74) Representative: Philips Intellectual Property & Standards
(86) International application number: PCT/EP2019/063707
(87) International publication number: WO 2019/233806

(56) References cited:
- US-A1- 2007 074 722
- US-A1- 2012 285 446
- US-A1- 2017 188 634

## Description

### FIELD OF THE INVENTION

The invention relates to systems for generating droplet outputs, such as nebulizers, and in particular to a method of verifying that a suitable cleaning procedure has been employed.

### BACKGROUND OF THE INVENTION

There are many applications in which generation of droplets, as a spray or mist, is required. These may make use of a droplet generator, which may become clogged over time. One application of particular interest is the delivery of medication in aerosol form.

There are three basic categories of device for delivering a medicament in aerosol form directly to the lungs of a patient during inhalation: metered-dose inhalers (MDI); dry powder inhalers (DPI); and nebulizers.

Generally, medicaments available for use in a MDI are provided in a pressurized formulation, medicaments available for use in a DPI are provided in a dry powder formulation, and medicaments available for use in a nebulizer are provided in an aqueous solution formulation.

This invention is of particular interest to nebulizers, although it is of interest more widely to systems for generating a droplet output. Due to their ease of use, many patients prefer nebulizers for the delivery of aerosolized medicaments. Nebulizers may employ different mechanisms for aerosolizing a medicament. For example, pneumatic (or jet) nebulizers employ compressed air or oxygen to disperse the liquid into aerosol form. An ultrasonic nebulizer, in contrast, employs an electrical source to excite a piezoelectric element which generates high frequency vibrations. These vibrations cause small droplets to separate from the surface of the medicament, thus forming an aerosol. Vibrating mesh technology (VMT) nebulizers employ a mesh having a number of microscopic apertures. A vibration is induced in the mesh (or in another component within the nebulizer) which causes the aqueous-based medicament to be extruded through the mesh apertures. The aqueous-based medicament is ejected from the mesh apertures in aerosol form.

VMT nebulizers have gained in popularity among patients. VMT nebulizers, however, possess some drawbacks. For example, some medicaments delivered by a VMT nebulizer tend to accumulate on the surface of the nebulizer mesh. This accumulation may occlude the microscopic apertures in the mesh, thereby reducing the effectiveness of the nebulizer. For example, total blockage of one or more apertures may decrease the efficiency of the device or ultimately prevent any medicament from being dispensed to the patient. In addition, partial blockage of one or more apertures may negatively affect the particle size or time of delivery, thus possibly reducing or eliminating the beneficial effects of the prescribed therapy.

It is therefore required to clean the mesh regularly of a VMT nebulizer. A typical nebulizer cleaning protocol requires that the nebulizer be disassembled and the mesh removed (either alone or as part of a module). A basic cleaning protocol involves washing mesh with warm soapy water. Afterwards, the mesh is rinsed in tap water and allowed to air dry. After the mesh has dried, it is re-inserted into the nebulizer which thereafter is reassembled for use. Other cleaning protocols have also been suggested for cleaning the mesh by soaking with cleaning agents and drying, or by boiling in water for a certain time followed by drying. Other cleaning protocols for example require the mesh to be placed in a high-temperature dishwasher, in an autoclave, and/or in an ultrasonic cleaner.

These cleaning protocols thus typically all require a time duration of at least several minutes. US 2007/0189919 discloses various cleaning operations for a VMT nebulizer.

The administration of therapy with a typical mesh nebulizer system can be quickly conducted, whereas the subsequent cleaning and drying operations are time consuming as explained above. The user is typically instructed by the manufacturer to clean regularly (i.e. as a minimum to rinse and air dry after use) or for disinfection to follow a set of instructions as discussed above.

There is therefore a risk that a user does not perform the required disassembly, cleaning and reassembly operations, through lack of time or interest.

US 2007/0074722 discloses a nebulizer which provides a reminder that cleaning is needed, if the device is not separated after use. However, this does not enable detection of whether cleaning has actually taken place. US 2012/0285446 discloses another nebulizer with releasably connected upper and lower portions. US 2017/0188634 discloses an aerosol generating device, in particular for a smoking article which generates an aerosol by heating. If a smoking article is removed from the device. The heating element may then be subjected to a cleaning regime.

There remains a need to assess whether or not correct cleaning has taken place, so that correct operation of the nebulizer can be maintained.

### SUMMARY OF THE INVENTION

The invention is defined by the claims.

According to examples in accordance with an aspect of the invention, there is provided a system for generating a droplet output, comprising:
a main body;
a droplet generator, wherein the droplet generator is separable from the main body for cleaning;
a sensor for detecting separation of the droplet generator from the main body;
a timer; and
a controller, which is adapted to:
   control the timer to determine a separation time duration during which the droplet generator is separated from the main body; and
   generate advisory information for output to a user of the system relating to cleaning of the droplet generator, and which information takes into account the determined separation time duration.

This system detects when the droplet generator is separated for cleaning, and also monitors a time duration of that separation. The separation time duration must be more than a certain threshold in order for the recommended cleaning protocol to have been followed, so the device is able to provide a warning when it is determined that the correct cleaning protocol cannot have been followed.

The controller may be adapted to set a threshold time period, and is adapted to generate advisory information to indicate that correct cleaning has not been conducted if the determined separation time duration is less than the threshold time period.

The threshold time period is for example longer than the time that would be required to perform a cursory rinse, but shorter than the typical time that would be required to perform the full recommended cleaning protocol. Thus, it enables discrimination between a cursory rinse and a full wash.

The threshold time period is for example at least 5 minutes, for example at least 10 minutes.

The controller is preferably adapted to determine a separation time duration during which the droplet generator is separated from the main body either since the last use of the system or after a set number of uses of the system.

The device will thus be set to the recommended cleaning protocol. This may be a rinse after each use, but a full clean after a fixed number of uses, or it may be a full clean after each use. Thus, generally, the device preferably monitors when the recommended cleaning protocol is required based on usage information as well as the recommended cleaning schedule.

The device may also monitor a time since the last detected full clean, so that regardless of use, a clean is recommended after a fixed time period.

The system may further comprise a timer system for timing a dose delivery time period, and wherein the controller is adapted to generate the advisory information further taking into account the dose delivery time period.

The time taken for a dose delivery depends on the state of cleanliness. Thus, after a cleaning operation, it is expected that there is a step change in the dose delivery time period. This provides a way to determine that actual cleaning has taken place, in addition to detecting device separation.

The timing system for example comprises a dry detection system for detecting an end of dose, and a timer. By timing until a dry state is detected, the dose delivery time is obtained.

The system may comprise a driver for providing a drive signal to the droplet generator, wherein the dry detection system comprises a system for monitoring characteristics of the drive signal or electrical characteristics of the droplet generator. The drive signal may be a drive current. This provides one way to implement dry detection.

A liquid level sensor may be provided for determining when a liquid sample has been converted into droplets. Thus, the timing may be carried out from the start of a droplet delivery period to the point when the liquid level reaches a fixed point (which may or may not be delivery of the full sample). Liquid level detection provides another way to implement dry detection.

In all cases, when the droplet generator is partially clogged, the generation of a droplet output (e.g. for atomization of a fixed dose of medication) takes longer. Thus, the additional monitoring of the operation time provides a further indication of the state of cleanliness of the droplet generator, and this additional information may be used to make the cleaning advice more accurate.

The system may comprise a vibrating mesh nebulizer and the droplet generator comprises a nebulizer mesh. The liquid sample is then a dose of a medicament.

In this case, the nebulizer mesh may be part of a mouthpiece, and the sensor is for detecting separation of the mouthpiece from the main body. The mouthpiece may be subjected to the cleaning protocol as a whole, or it may require further disassembly.

To output the advisory information to the user may comprise to output the information to the user via the system itself or to output the information to the user via a device external to the system.

According to an embodiment, the system comprises a wireless component for wirelessly transmitting the advisory information for output to a user of the system. For example, the wireless component, e.g. a chip, is configured for transmitting the advisory information to a device external from the system, e.g. a smartphone or a wearable. For example, a smartphone receives the advisory information whereby an app running on the smartphone displays the advisory information to the user in a suitable manner via the display of the smartphone.

According to an embodiment, the system comprises a display, e.g. a screen. In that embodiment, the advisory information is output to the user via the display.

According to an embodiment, the system comprises an audible output, e.g. a loudspeaker. In that embodiment, the advisory information is output to the user via this audible output. For different advisory information, different sounds may be used. For this, the audible output is capable of generating different sounds.

The invention also provides a method for monitoring cleaning of a system for generating a droplet output which comprises a droplet generator and a main body which are separable for cleaning of the droplet generator, the method being implemented by the system, and comprising:
detecting separation of the droplet generator from the main body;
determining a separation time duration during which the droplet generator is separated from the main body; and
generating and outputting advisory information to a user of the system relating to cleaning of the droplet generator, which information takes into account the determined separation time duration.

The method for example comprises setting a threshold time period, and generating advisory information to indicate that correct cleaning has not been conducted if the determined separation time duration is less than the threshold time period.

The method may also comprise determining an operation time for generating the droplet output and generating the advisory information further takes into account the determined operation time.

The operation time may be obtained based on detecting when the droplet generator becomes dry thereby signaling the end of a dose delivery, and timing to this point in time.

According to an embodiment, outputting the advisory information to a user of the system comprises wirelessly transmitting the advisory information to the user. For example, the information may be transmitted to an intermediate server that communicates with a device that outputs the information to the user. Alternatively, the information is directly transmitted to a device, e.g. a smartphone, that outputs the information to the user.

According to an embodiment, outputting the advisory information to a user of the system comprises displaying the advisory information to the user via a display of the system.

According to an embodiment, outputting the advisory information to a user of the system comprises generating a sound related to the advisory information using an audible output of the system. For different advisory information, different sounds may be used.

The system preferably comprises a vibrating mesh nebulizer and the droplet generator comprises a nebulizer mesh. The mesh is preferably part of a mouthpiece, and detecting separation of the mesh from the main body comprises detecting separation of the mouthpiece from the main body.

The invention may be implemented at least in part in software, and the invention thus also provides a computer program comprising computer program code means which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known vibrating mesh nebulizer;
Figure 2 shows the nebulizer of Figure 1 as an exploded view;
Figure 3 shows in schematic form a nebulizer in accordance with an example of the invention; and
Figure 4 shows a cleaning monitoring method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a droplet generating system which determines a separation time duration during which a droplet generator is separated from a main body of the system. This timing information is used to generate advisory information relating to cleaning of the droplet generator. In particular, it is possible to provide a warning when it is detected that a cleaning protocol has not been followed.

The invention is of particular interest for vibrating mesh nebulizers. The general construction and operation of a vibrating mesh nebulizer will first be described, before the modification in accordance with the invention is explained.

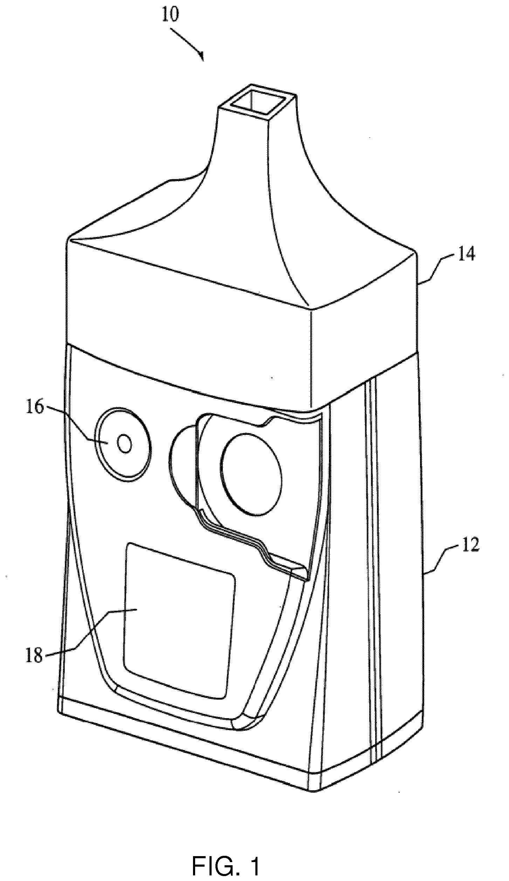

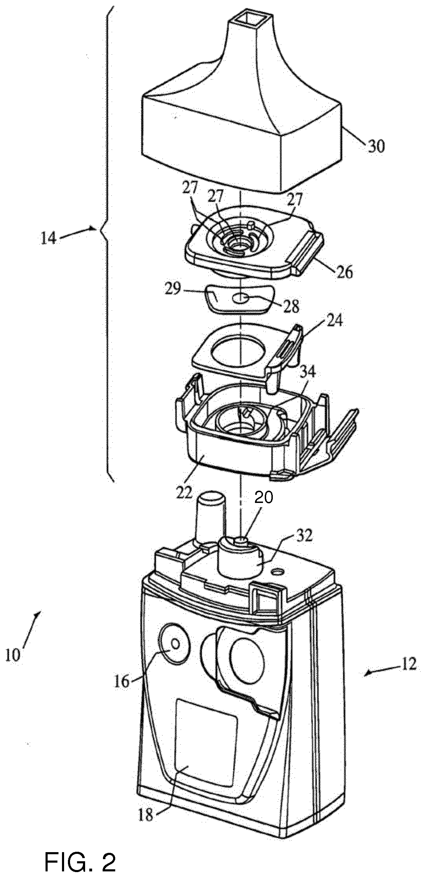

Figure 1 is an isometric view and Figure 2 is an exploded view, of a VMT nebulizer 10. These images are taken from US 2007/0189919.

The nebulizer 10 includes a lower portion in the form of a main body 12 and an upper portion in the form of a mouthpiece 14. The main body 12 includes a power supply source (not shown) such as a rechargeable battery pack, an on/off switch 16, a display 18, and a horn device 20. The main body 12 houses circuitry (not shown) used to control the operation of the nebulizer 10. In the exemplary embodiment, the circuitry includes a microprocessor which is structured to access instructions and control data stored, for example, on a disc and/or a memory. The instructions may include the dosage amount, the dosing frequency, and the number of doses that may be delivered. The control data may include the medicament lot number and expiration date. The microprocessor is adapted to execute the routines in response to the instructions and control data and, in conjunction with the other circuitry, cause the nebulizer 10 to dispense the proper dose of medicament to a patient.

The mouthpiece 14 is removably connected to the main body 12, and includes a medicament chamber 22, a guide 24, a flexible housing 29, a chamber lid 26, and a mouthpiece housing 30. The horn device 20 extends from a boss 32 which fits into opening 34 of the chamber 22. The chamber 22 is structured to hold an aqueous-based medicament therein. The chamber 22 along with guide 24 directs the medicament towards horn device 20. The flexible housing 29 holds a mesh 28 having an upper surface and a lower surface.

The horn device 20 includes a piezoelectric element (not shown) that is in electrical communication with the circuitry (not shown) contained within the main body 12. The horn 20 and piezoelectric element of the main body 12 vibrates the mesh 28 of to form a nebulization mechanism. More specifically, the lower surface of the mesh 28 contacts a top surface of the horn 20. The piezoelectric element is structured to oscillate when driven by the circuitry contained within the main body 12. A vibratory motion caused by the oscillation of the piezoelectric element is transferred to the horn 20 and to the mesh 28. The vibratory motion causes the aqueous-based medicament within chamber 22 to pass through a number of apertures of the mesh 28. The medicament leaving the apertures at the upper surface of the mesh 28 is aerosolized.

After exiting the apertures of the mesh, the aerosolized medicament passes from the upper surface of mesh 28 through openings 27 toward the exit of the mouthpiece 14. The aerosolized medicament can then be inhaled by a patient.

The mouthpiece 14 of the nebulizer 10 can be disassembled into its component parts, for example, during cleaning. Particularly, the chamber lid 26 and mesh 28 may be separated from the other components of the nebulizer 10. The mesh 28 may remain housed within the chamber lid 26 during cleaning and/or the mesh 28 may be removed from the chamber lid 26 and cleaned separately.

This is just one example of possible nebulizer architecture, and others are possible.

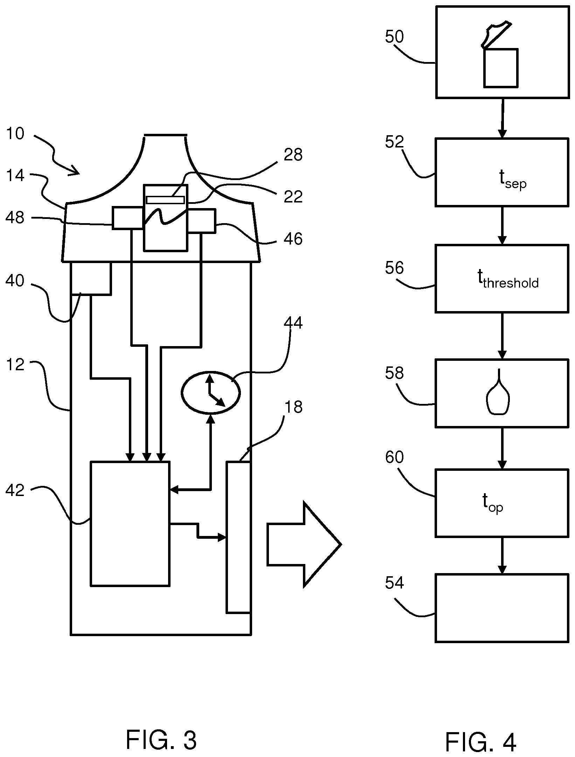

Figure 3 shows a vibrating mesh nebulizer in schematic form, to illustrate the additional features to the known system of Figures 1 and 2.

The nebulizer 10 again comprises a main body 12 and a mouthpiece 14 which includes a nebulizer mesh 28. The nebulizer mesh 28 is separable from the main body 12 for cleaning.

A sensor 40 is provided for detecting separation of the nebulizer mesh from the main body 12, in particular by detecting separation of the mouthpiece 14 from the main body 12.

The sensor may comprise a contact sensor, whereby electrical contact is made when the mouthpiece and main body are connected. Other sensors may be used, such an optical sensor (e.g. an LED and a photodiode for detecting reflection from the mouthpiece when it is connected) or any other contact or proximity sensor.

The main body has a controller 42 which receives the information from the sensor 40. The controller also includes a drive signal generator for generating a drive signal for the mesh 48. This drive signal for example has a frequency chosen to match a resonant frequency of the mesh which is typically of the order of magnitude of 100kHz such as 128kHz. A drive current is provided to the mesh 28 to set it into resonance.

A timer 44 is used for determining time periods, under the control of the controller 42. The timer 44 is operated to measure a separation time duration (simply referred to as a separation time below) during which the nebulizer mesh, i.e. the mouthpiece, is separated from the main body. According to an embodiment, advisory information is then generated by the controller for output to a user of the nebulizer using the display 18 (and/or an audible output, e.g. a loudspeaker). According to an embodiment, the advisory information is generated by the controller for output to a user of the nebulizer whereby the advisory information is transferred wirelessly for output to the user. This output provides information relating to cleaning of the nebulizer mesh. In particular, it provides a warning that correct cleaning is overdue and/or an instruction to perform the required cleaning task. This warning or instruction takes into account the determined separation time.

According to an embodiment, the system comprises a wireless component for wirlessly transmitting the advisory information. For example, the system comprises a wireless chip capable of transmitting the advisory information to another device, e.g. a wearable device or a smartphone. The wireless component may be a zigbee/bluetooth/wifi (or any other wireless protocol) chip capable of: 1) transmiting the advisory data either directly to a device capable of outputting the advisory information to the user; or 2) transmiting the advisory data to an intermediate device, e.g. a server, that communicates with a device capable of outputting the advisory information to the user.

The separation time needs to be more than a certain threshold in order for the recommended cleaning protocol to have been followed, so the device is able to provide a warning when it is determined that the correct cleaning protocol cannot have been followed.

The threshold is longer than the time that would be required to perform a cursory rinse but shorter than the typical time that would be required to perform the full recommended cleaning protocol. Thus, it enables discrimination between a cursory rinse and a full wash. The threshold time period is for example at least 5 minutes, for example at least 10 minutes. It will be set to a value which depends on the recommended cleaning procedure, since that procedure will require a minimum time period to be completed correctly.

The cleaning protocol being monitored may be required after each use of the nebulizer, so the monitoring takes place after each use. However, the cleaning protocol may instead require a rinse after each use and a complete clean after a set number of uses or after a set time such as weekly. Thus, the threshold may be applied either each time the mesh is separated from the main body or only after a set number of uses of the nebulizer or set period of time.

There may be multiple thresholds. For example it may be required that there is separation after each use to ensure that at least a rinse has been carried out. This implies a short time threshold, e.g. 1 minute. A longer time threshold may then be applied when a full wash has been required, e.g. 10 minutes.

Different cleaning protocols are possible, for example depending on the condition being managed/treated. Thus, the monitoring of the cleaning takes account of the specified cleaning protocol.

To the extent described above, the system is able to detect failure to have conducted the required cleaning procedure. Additional sensing may be used to provide greater confidence that the required cleaning has been carried out.

A first effect of cleaning is that the dose delivery time changes. Thus, Figure 3 shows a timing system 44, 46 for measuring the dose delivery time and the controller 42 is then adapted to generate the advisory information further taking into account the dose delivery time, which provides a positive indication that cleaning has actually taken place.

The timing system 44, 46 for example measures a time from when the dose begins to when the mesh has become dry, and thus includes a dry detection system 46. detecting an end of dose. The timer 44 described above may form part of the timing system 44, 46. In one example, the dry detection system 46 can be implemented by monitoring a drive current to the mesh 28. The mesh drive signal brings the mesh into resonance, and the load presented by the mesh influences the required drive current. This load depends on the wet or dry state of the mesh, so that a change (drop) in current is observed when the mesh is dry, at the end of the dose delivery.

In this way, changes in the operation time for nebulizing a dose of drug after the device has been reconnected will reflect that the mesh has indeed been cleaned. It is known that the operation time is dependent on the state of cleanliness of the mesh, and indeed it is known to require a user to measure this time to verify the correct functioning of the nebulizer.

The monitoring of the drive current to distinguish between wet and dry states is discussed in more detail in WO 2015/010809.

The dose delivery time after a full cleaning event should return to a baseline value. Thus, the dose delivery time may be used not only to confirm that cleaning has taken place, but also to provide an indication of the effectiveness of the cleaning.

Thus, an additional alert may be provided to indicate that, even though cleaning seems to have taken place (based on the separation time), the cleaning has not been effective (based on the dose time not returning to the expected baseline value or with a threshold difference). This may either indicate the end of life of the mesh or it may indicate that the correct cleaning protocol was not followed.

The current monitoring provides one way to detect a dry mesh at the end of the delivered dose, and thereby enable the dose delivery time to be measured. An alternative approach is to monitor the quantity of liquid, and a liquid level sensor 48 may be provided for determining when the dose, or a fixed portion of the dose, of drug to be delivered to the user has been atomized. Thus, the timing may be carried out from the start of a dose delivery to the point when the liquid level reaches a fixed point (which may or may not be delivery of the full dose) or the timing may be carried out from the start of a dose delivery and a detection of a dry state based on monitoring the drive current to the mesh (or monitoring other electrical characteristics, such as measuring an impedance).

A liquid level sensor for example may be implemented as liquid sensing electrodes at a certain location within the dose chamber which detect the presence or absence of liquid based on a change in capacitance or resistance. Any suitable liquid sensing or level sensing approach may be used.

Figure 5 shows a method for monitoring cleaning of a system for generating a droplet output. The method comprises:
in step 50, detecting separation of a droplet generator from a main body;
in step 52 determining a separation time (tₛₑₚ) during which the droplet generator is separated from the main body; and
in step 54 generating and outputting advisory information to a user of the system relating to cleaning of the droplet generator, which information takes into account the determined separation time.

The method for example also comprises in step 56 setting a threshold time (threshold) period and in step 58 detecting when the droplet generator has become dry at the end of a dose delivery.

The method may also comprise in step 60 determining an operation time (tₒₚ) for generating the droplet output and generating the advisory information further takes into account the determined operation time.

As is clear from the description above, the method is preferably implemented by a vibrating mesh nebulizer, and the operation time is the time to atomize a fixed dose (or proportion thereof) of a liquid medicament.

The invention may be applied to other droplet generating systems which have a cleaning protocol for the droplet generator. Examples are delivery systems for pesticides, disinfection, painting, scent delivery or spray cooling systems.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system for generating a droplet output, comprising:
a main body (12);
a droplet generator (28), wherein the droplet generator is separable from the main body for cleaning;
a sensor (40) for detecting separation of the droplet generator from the main body;
a timer (44);
**characterized in that** the system comprises:
a controller (42), which is adapted to:
control the timer (44) to determine a separation time duration during which the droplet generator is separated from the main body; and
generate advisory information for output to a user of the system relating to cleaning of the droplet generator, and which information takes into account the determined separation time duration.

2. A system as claimed in claim 1, wherein the controller (42) is adapted to set a threshold time period, and is adapted to generate advisory information to indicate that correct cleaning has not been conducted if the determined separation time duration is less than the threshold time period.

3. A system as claimed in claim 2, wherein the threshold time period is at least 5 minutes, for example at least 10 minutes.

4. A system as claimed in any one of claims 1 to 3, wherein the controller is (42) adapted to determine a separation time duration during which the droplet generator is separated from the main body since:
the last use of the system; or
a set number of uses of the system.

5. A system as claimed in any one of claims 1 to 4, further comprising a timer system (44, 46) for timing a dose delivery time period, and wherein the controller is adapted to generate the advisory information further taking into account the dose delivery time period.

6. A system as claimed in claim 5, wherein the timing system (44, 46) comprises a dry detection system (46) for detecting an end of dose and a timer (44).

7. A system as claimed in claim 6, comprising a driver for providing a drive signal to the droplet generator, wherein the dry detection system (46) comprises a system for monitoring characteristics of the drive signal or electrical characteristics of the droplet generator.

8. A system as claimed in any one of claims 1 to 7, wherein the system comprises a vibrating mesh nebulizer and the droplet generator (28) comprises a nebulizer mesh.

9. A system as claimed in claim 8, wherein the nebulizer mesh (28) is part of a mouthpiece (14), wherein the sensor is for detecting separation of the mouthpiece from the main body.

10. A system as claimed in any of the preceding claims, further comprising a wireless component for wirelessly transmitting the advisory information for output to a user of the system.

11. A system as claimed in any of claims 1 to 9, further comprising a display (18); and wherein the advisory information is output to the user via the display (18).

12. A system as claimed in any of claims 1 to 9, further comprising an audible output; and wherein the advisory information is output to the user via the audible output.

13. A system as claimed in any of the preceding claims, wherein the determined separation time duration is used to generate the advisory information.

14. A method for monitoring cleaning of a system for generating a droplet output which comprises a droplet generator (28) and a main body (12) which are separable for cleaning of the droplet generator, the method being implemented by the system and comprising:
(50) detecting separation of the droplet generator from the main body; **characterized in that** the method comprises:
(52) determining a separation time duration during which the droplet generator is separated from the main body; and
(54) generating and outputting advisory information to a user of the system relating to cleaning of the droplet generator, which information takes into account the determined separation time duration.

15. A method as claimed in claim 14, comprising (56) setting a threshold time period, and generating advisory information to indicate that correct cleaning has not been conducted if the determined separation time duration is less than the threshold time period.

16. A method as claimed in claim 15, further comprising (60) determining an operation time for generating the droplet output and generating the advisory information further takes into account the determined operation time.

17. A method as claimed in claim 16, comprising (58) detecting when the droplet generator becomes dry thereby signaling the end of a dose delivery to enable the operation time to be determined.

18. A method as claimed in any one of claims 14 to 17, wherein the system comprises a vibrating mesh nebulizer and the droplet generator comprises a nebulizer mesh (28).

19. A method as claimed in any of claims 14 to 18, wherein outputting the advisory information to a user of the system comprises wirelessly transmitting the advisory information to the user.

20. A method as claimed in any of claims 14 to 18, wherein outputting the advisory information to a user of the system comprises displaying the advisory information to the user.

21. A method as claimed in any of claims 14 to 18, wherein outputting the advisory information to a user of the system comprises generating a sound related to the advisory information.

22. A method as claimed in any of claims 14 to 21, wherein the determined separation time duration is used to generate the advisory information.

## Patentansprüche

1. Ein System zum Erzeugen einer Tröpfchenausgabe, das Folgendes umfasst:
ein Hauptgehäuse (12);
einen Tröpfchengenerator (28), wobei der Tröpfchengenerator zum Reinigen vom Hauptgehäuse getrennt werden kann;
einen Sensor (40) zum Erkennen des Trennens des Tröpfchengenerators vom Hauptgehäuse;
einen Zeitgeber (44);
**dadurch gekennzeichnet, dass** das System Folgendes umfasst:
eine Steuerung (42), die folgende Schritte durchführt:
Steuern des Zeitgebers (44), um die Trennungsdauer zu ermitteln, über die der Tröpfchengenerator vom Hauptgehäuse getrennt ist; und
Generieren von unterstützenden Daten, die an einen Benutzer des Systems ausgegeben werden, und die sich auf das Reinigen des Tröpfchengenerators beziehen,
wobei die Daten die ermittelte Trennungsdauer umfassen.

2. Ein System gemäß Anspruch 1, wobei die Steuerung (42) einen Schwellenzeitraum festlegt und unterstützende Daten generiert, um anzuzeigen, dass keine ordnungsgemäße durchgeführt wurde, wenn die ermittelte Trennungsdauer kürzer als der Schwellenzeitraum ist.

3. Ein System gemäß Anspruch 2, wobei der Schwellenzeitraum mindestens 5 Minuten und somit beispielsweise mindestens 10 Minuten beträgt.

4. Ein System gemäß einem der Ansprüche 1 bis 3, wobei die Steuerung (42) die Trennungsdauer ermittelt, über die der Tröpfchengenerator vom Hauptgehäuse getrennt ist, seit
der letzten Verwendung des Systems; oder
einer bestimmten Anzahl von Verwendungen des Systems.

5. Ein System gemäß einem der Ansprüche 1 bis 4, das zudem ein Zeitgebersystem (44, 46) für das zeitliche Steuern des Dosisabgabezeitraums umfasst, und wobei die Steuerung die unterstützenden Daten zudem unter Berücksichtigung des Dosisabgabezeitraums generiert.

6. Ein System gemäß Anspruch 5, wobei das Zeitgebersystem (44, 46) ein Trockenerkennungssystem (46) zum Erkennen des Ablaufs einer Dosis sowie einen Zeitgeber (44) umfasst.

7. Ein System gemäß Anspruch 6, das ein Stellglied zum Bereitstellen eines Stellsignals für den Tröpfchengenerator umfasst, wobei das Trockenerkennungssystem (46) ein System zum Überwachen der Eigenschaften des Stellsignals oder der elektrischen Eigenschaften des Tröpfchengenerators umfasst.

8. Ein System gemäß einem der Ansprüche 1 bis 7, wobei das System einen vibrierenden Siebzerstäuber und der Tröpfchengenerator (28) ein Zerstäubersieb umfasst.

9. Ein System gemäß Anspruch 8, wobei das Zerstäubersieb (28) Teil eines Mundstücks (14) ist, und wobei der Sensor das Trennen des Mundstücks vom Gehäuse erkennt.

10. Ein System gemäß einem der vorherigen Ansprüche, das zudem eine Drahtlos-Komponente zum drahtlosen Übertragen der unterstützenden Daten für die Ausgabe an einen Benutzer des Systems umfasst.

11. Ein System gemäß einem der Ansprüche 1 bis 9, das zudem eine Anzeige (18) umfasst; und wobei die unterstützenden Daten über die Anzeige (18) an den Benutzer ausgegeben werden.

12. Ein System gemäß einem der Ansprüche 1 bis 9, das zudem eine akustische Ausgabe umfasst; und wobei die unterstützenden Daten über die akustische Ausgabe an den Benutzer ausgegeben werden.

13. Ein System gemäß einem der vorherigen Ansprüche, wobei die ermittelte Trennungsdauer verwendet wird, um die unterstützenden Daten zu erzeugen.

14. Eine Methode zum Überwachen der Reinigung eines Systems zum Erzeugen einer Tröpfchenausgabe, das einen Tröpfchengenerator (28) und ein Hauptgehäuse (12) umfasst, die zum Reinigen des Tröpfchengenerators getrennt werden können, wobei die Methode vom System umgesetzt wird und folgende Schritte umfasst:
(50) Erkennen des Trennens des Tröpfchengenerators vom Hauptgehäuse; **dadurch gekennzeichnet, dass** die Methode folgende Schritte umfasst:
(52) Ermitteln der Trennungsdauer, über die der Tröpfchengenerator vom Hauptgehäuse getrennt ist; und
(54) Generieren von unterstützenden Daten, die an einen Benutzer des Systems ausgegeben werden, und die sich auf das Reinigen des Tröpfchengenerators beziehen, wobei die Daten die ermittelte Trennungsdauer umfassen.

15. Eine Methode gemäß Anspruch 14, die das (56) Festlegen eines Schwellenzeitraums sowie das Generieren unterstützender Daten umfasst, um anzuzeigen, dass keine ordnungsgemäße durchgeführt wurde, wenn die ermittelte Trennungsdauer kürzer als der Schwellenzeitraum ist.

16. Eine Methode gemäß Anspruch 15, die zudem das Ermitteln (60) der Betriebszeit zum Erzeugen der Tröpfchenausgabe umfasst, wobei die ermittelte Betriebszeit beim Generieren der unterstützenden Daten berücksichtigt wird.

17. Eine Methode gemäß Anspruch 16, Im Rahmen derer zudem (58) erkannt wird, wann der Tröpfchengenerator trocken ist, um das Ablaufen einer Dosisabgabe zu signalisieren und die Betriebszeit bestimmen zu können.

18. Eine Methode gemäß einem der Ansprüche 14 bis 17, wobei das System einen vibrierenden Siebzerstäuber und der Tröpfchengenerator ein Zerstäubersieb (28) umfasst.

19. Die Methode gemäß einem der Ansprüche 14 bis 18, wobei das Ausgeben der unterstützenden Daten an einen Benutzer des Systems das drahtlose Übertragen der unterstützenden Daten an den Benutzer umfasst.

20. Die Methode gemäß einem der Ansprüche 14 bis 18, wobei das Ausgeben der unterstützenden Daten an einen Benutzer des Systems das Anzeigen der unterstützenden Daten für den Benutzer umfasst.

21. Die Methode gemäß einem der Ansprüche 14 bis 18, wobei das Ausgeben der unterstützenden Daten an einen Benutzer des Systems das Erzeugen eines auf die unterstützenden Daten verweisenden Tons umfasst.

22. Die Methode gemäß einem der Ansprüche 14 bis 21, wobei die ermittelte Trennungsdauer verwendet wird, um die unterstützenden Daten zu erzeugen.

## Revendications

1. Système de génération d'une sortie de gouttelette, comprenant :
un corps principal (12) ;
un générateur de gouttelette (28), dans lequel le générateur de gouttelette peut se séparer du corps principal pour le nettoyage ;
un capteur (40) permettant de détecter la séparation du détecteur de gouttelette du corps principal ;
un minuteur (44) ;
**caractérisé en ce que** le système comprend :
un contrôleur (42), qui est conçu pour :
contrôler le minuteur (44) afin de déterminer une durée de temps de séparation durant lequel le générateur de gouttelette est séparé du corps principal ; et
générer des informations de conseil pour aider un utilisateur du système à nettoyer le générateur de gouttelettes, ces informations prenant en compte la durée de temps de séparation déterminée.

2. Système selon la revendication 1, dans lequel le contrôleur (42) est conçu pour définir une période de temps seuil et pour générer des informations de conseil indiquant que le nettoyage approprié n'a pas été réalisé si la durée de temps de séparation déterminée est inférieure à la période de temps seuil.

3. Système selon la revendication 2, dans lequel la période de temps seuil est d'au moins 5 minutes, par exemple d'au moins 10 minutes.

4. Système selon l'une quelconque des revendications 1 à 3, dans lequel le contrôleur est (42) conçu pour déterminer une durée de temps de séparation durant laquelle le générateur de gouttelette est séparé du corps principal depuis :
la dernière utilisation du système ; ou
un nombre défini d'utilisations du système.

5. Système selon l'une quelconque des revendications 1 à 4, comprenant en outre un système de minuterie (44, 46) pour minuter une période de temps d'administration d'une dose, et dans lequel le contrôleur est conçu pour générer des informations de conseil en prenant en compte en outre la période de temps d'administration d'une dose.

6. Système selon la revendication 5, dans lequel le système de minuterie (44, 46) comprend un système de détection sec (46) pour détecter une fin de dose et un minuteur (44).

7. Système selon la revendication 6, comprenant un entraîneur permettant de fournir un signal d'entraînement au générateur de gouttelette, dans lequel le système de détection sec (46) comprend un système pour surveiller les caractéristiques du signal d'entraînement ou les caractéristiques électriques du générateur de gouttelette.

8. Système selon l'une quelconque des revendications 1 à 7, dans lequel le système comprend un nébuliseur à mailles vibrant et le générateur de gouttelette (28) comprend une maille de nébuliseur.

9. Système selon la revendication 8, dans lequel le nébuliseur à mailles vibrant (28) fait partie d'un embout buccal (14), dans lequel le capteur est conçu pour détecter la séparation de l'embout buccal du corps principal.

10. Système selon l'une quelconque des revendications précédentes, comprenant en outre un composant sans fil pour la transmission sans fil d'informations de conseil à un utilisateur du système.

11. Système selon l'une quelconque des revendications 1 à 9, comprenant en outre un écran (18) ; et dans lequel les informations de conseil sont transmises à l'utilisateur via l'écran (18).

12. Système selon l'une quelconque des revendications 1 à 9, comprenant en outre une sortie audible ; et dans lequel les informations de conseil sont transmises à l'utilisateur via la sortie audible.

13. Système selon l'une quelconque des revendications précédentes, dans lequel la durée de temps de séparation déterminée est utilisée pour générer des informations de conseil.

14. Procédé pour surveiller le nettoyage d'un système de génération de sortie de gouttelette qui comprend un générateur de gouttelette (28) et un corps principal (12) qui peuvent se séparer pour le nettoyage du générateur de gouttelette, le procédé étant mis en œuvre par le système et comprenant :
(50) la détection de la séparation du générateur de gouttelette du corps principal ;
**caractérisé en ce que** le procédé comprend :
(52) la détermination d'une durée de temps de séparation durant lequel le générateur de gouttelette est séparé du corps principal ; et
(54) la génération et la transmission d'informations de conseil à un utilisateur du système pour le nettoyage du générateur de gouttelette, lesdites informations prenant en compte la durée de temps de séparation déterminée.

15. Procédé selon la revendication 14, comprenant (56) la fixation d'une période de temps seuil, et la génération d'informations de conseil pour indiquer que le nettoyage approprié n'a pas été réalisé si la durée de temps de séparation déterminée est inférieure à la période de temps seuil.

16. Procédé selon la revendication 15, comprenant (60) en outre la détermination d'un temps de fonctionnement pour générer la sortie de gouttelette et la génération d'informations de conseil prend en outre en compte le temps de fonctionnement déterminé.

17. Procédé selon la revendication 16, comprenant (58) la détection du moment où le générateur de gouttelette devient sec signalant ainsi la fin d'une administration de dose pour permettre au temps de fonctionnement d'être déterminé.

18. Procédé selon l'une quelconque des revendications 14 à 17, dans lequel le système comprend un nébuliseur à mailles vibrant et le générateur de gouttelette comprend une maille de nébuliseur (28).

19. Procédé selon l'une quelconque des revendications 14 à 18, dans lequel la transmission des informations de conseil à un utilisateur du système comprend la transmission sans fil des informations de conseil à l'utilisateur.

20. Procédé selon l'une quelconque des revendications 14 à 18, dans lequel la transmission des informations de conseil à un utilisateur du système comprend l'affichage des informations de conseil à l'utilisateur.

21. Procédé selon l'une quelconque des revendications 14 à 18, dans lequel la transmission des informations de conseil à un utilisateur du système comprend la génération d'un son relatif aux informations de conseil.

22. Procédé selon l'une quelconque des revendications 14 à 21, dans lequel la durée de temps de séparation déterminée est utilisée pour générer des informations de conseil.
